# EUROPÄISCHE PATENTSCHRIFT

(11) **EP 1 841 398 B1**
(45) Veröffentlichungstag und Bekanntmachung des Hinweises auf die Patenterteilung: **19.11.2014**
(21) Anmeldenummer: 06707807.1
(22) Anmeldetag: 19.01.2006
(51) Int. Cl.: A61K 6/02, A61K 6/027, A61K 6/00, A61K 6/033

(54) **BESCHICHTETE DENTALPULVER**
COATED DENTAL POWDER
POUDRE DENTAIRE RECOUVERTE

(30) Priorität: 27.01.2005 DE 102005003755
(43) Veröffentlichungstag der Anmeldung: 10.10.2007
(73) Patentinhaber: Ivoclar Vivadent AG, 9494 Schaan (LI)
(72) Erfinder: HÖLAND, Wolfram, CH-9494 Schaan (LI); RITZBERGER, Christian, CH-9472 Grabs (CH); MOSZNER, Norbert, FL-9495 Triesen (LI); KERSCHBAUMER, Harald, A-6833 Klaus (AT); RHEINBERGER, Volker, CH-9490 Vaduz (LI); DELLAGIACOMO, Ricardo, A-6805 Feldkirch-Gisingen (AT)
(74) Vertreter: Fitzner, Uwe
(86) Internationale Anmeldenummer: PCT/EP2006/050389
(87) Internationale Veröffentlichungsnummer: WO 2006/079619

(56) Entgegenhaltungen:
- DE-A1- 19 508 586
- US-A1- 2004 224 087
- DATABASE WPI Section Ch, Week 199506 Derwent Publications Ltd., London, GB; Class A96, AN 1995-040960 XP002377395 & JP 06 321515 A (OSAKA CEMENT KK) 22. November 1994 (1994-11-22)
- DATABASE WPI Section Ch, Week 198329 Derwent Publications Ltd., London, GB; Class A96, AN 1983-714751 XP002377396 & JP 58 099406 A (TOKUYAMA SODA KK) 13. Juni 1983 (1983-06-13)

## Beschreibung

Die vorliegende Erfindung betrifft ein neuartiges Dentalpulver, sowie ein Verfahren zu dessen Herstellung.

Nach dem Stand der Technik sind Beschichtungen von Gläsern, Glaskeramiken oder Keramiken mit organischen Werkstoffen bekannt. Diese Beschichtungen werden als Dick- oder Dünnschichten bezeichnet. Die Dünnschichten weisen Stärken von Nanometerdimension bis Mikrometerdimension auf. Der Stoffmengenanteil dieser organischen Beschichtungskomponenten im Vergleich zum Substratmaterial reicht von 0.2 Ma.-% bis ca. 5 Ma.-%. Typische Beispiele für derartige Dünnbeschichtungen sind das schlichte Aufbringen auf Glasfasern, das Aufbringen von Sol-Gel-Schichten auf Gläser (Schmidt, H., J. Non-Cryst. Sol. 178, 1994, 302) oder das Aufbringen von Presshilfsmitteln auf Keramikpulver zur Herstellung von hochfesten Keramiken, z.B. ZrO₂.

Dünnbeschichtungen von monolithischen Körpern aus Metall oder Keramik werden von Tossatti et al. (Langmuir, 18, 2002,3537) vorgestellt. Bei diesen Beschichtungen handelt es sich um biokompatible organische Komponenten, die z.B. eine besondere Blutverträglichkeit aufweisen und auf Ti- Metall oder TiO₂ aufgebracht wurden. Beschichtungen von Pulvern wird nicht genannt.

Andererseits ist von Dentalpulvern, die zur Beschichtung von Substraten, z. B. von Metallgerüsten (z.B. einer Dentalkrone, oder einer mehrgliedrige Brücke) oder der Beschichtung von metallfreien Restaurationen z.B. aus Glaskeramik (Dentalkrone oder dreigliedrige Brücke) zur optimalen Verarbeitung der Pulver bekannt, dass Anmischflüssigkeiten verwendet werden müssen um hochviskose, dickflüssige Schlicker herzustellen. Diese Anmischflüssigkeiten erlauben es dem Zahntechniker aus dem Dentalpulver einen Schlicker mit optimaler Konsistenz herzustellen. Somit ist der Zahntechniker in der Lage, mit einem Pinsel die Masse auf die Suprastruktur (z.B. Krone, oder Brücke) aufzutragen und mit diesem Auftragevorgang die Form der Krone oder Brücke ideal zu modellieren. Die überschüssige Flüssigkeit wird abgesaugt oder mit einem Föhn entfernt. Anschließend wird die nächste Schicht Schlicker aufgebracht. Die Anmischflüssigkeiten stellen wässrige Medien mit geringen Anteilen an organischen und anorganischen Komponenten dar.

Nach diesem Beschichtungsvorgang der Substrate mit Schlicker und erster Entfernung der Flüssigkeit erfolgt die anschließende thermische Behandlung von Raumtemperatur bis zum Abschluss des Sintervorganges, der typischer Weise zwischen 700°C und 1000 °C liegt. Trotz der Verwendung von Anmischflüssigkeiten kommt es verschiedentlich zu Situationen, dass das Dentalpulver während der thermischen Behandlung nicht optimal auf dem Substrat haftet, so dass im Fertigprodukt kein optimaler Fügevorgang zwischen Dentalpulver und Substrat erreicht wird. Das wird dadurch sichtbar, dass bevorzugt im Approximalbereich einer Krone oder im Interdentalbereich zwischen einzelnen Kronensegmenten einer mehrgliedrigen Brücke ein Zurückziehen (englisch tearing) oder ein Abheben der Dentalkeramik von der Unterlage (Substrat) beobachtet wird. Tritt eine solche Situation auf, muss der Zahntechniker eine zusätzliche Schicht Schlicker aufbringen, um diesen entstandenen Spalt zu füllen.

Andererseits ist von pulverförmigen Dentalkeramiken, Dentalglaskeramiken oder Dentalgläsern bekannt, dass es während des Schwindungsvorganges im Prozess des Dichtsinterns zu unkontrollierten Reaktionen kommen kann, die die gewünschte Geometrie verändern (sog. Verziehen eines Formteiles).

In der DE 195 08 586 A1 wird die Verwendung von Partikeln als Füllstoff in Füllungskompositen beschrieben, die aus einem SiO₂-Kern bestehen und mit einem anderen Oxid beschichtet sind. Schwerpunkt in dieser Schrift ist die Herstellung von Kompositen mit bestimmten röntgenopaken Eigenschaften und einer einstellbaren Transluzenz. Die mögliche anschließende Beschichtung dieser "zweischichtigen" SiO₂-Partikel mit einer organischen Schicht dient der verbesserten Einarbeitung dieser Füllstoffe in die organische Matrix des Füllungskomposits.

Die DE 197 41 286 A1 beschreibt kugelförmige Partikel, die aus einem SiO₂-Kern und/oder weiteren Oxiden bestehen und eine bestimmte Größenordnung aufweisen. Zusätzlich sind diese mit einem polymerisierbaren Bindemittel überzogen, wobei dieser Überzug eine kovalente Einbindung dieser Partikel in die Polymermatrix ermöglicht und auf diese Weise eine hohe Festigkeit des organisch-anorganischen Verbundes im Komposit gewährleistet.

Die US 2004/0224087 A1 beschreibt die Herstellung und Verwendung von Mischoxidpartikeln, die aus mehreren Lagen unterschiedlicher Oxide bestehen als Füllstoff in Dentalkompositen, wobei eine der äußeren Lagen der Partikel im wesentlichen aus SiO₂ aufgebaut ist. Die Oxide des Kerns der Partikel sind aus verschiedenen Metallen aufgebaut und weisen einen höheren Brechungsindex auf als SiO₂ allein.

Auch die US 2004/0134230 A1 beschreibt die Herstellung von Füllstoffen für Komposite. Dabei weisen diese Glaspulver bestimmte chemische Eigenschaften und Morphologien auf. In einem Falle werden diese Partikel auch mit organischen Materialien beschichtet, wobei die Aufgabe dieser Beschichtung, der besseren Einbindung dieser Partikel in die Harzmatrix dient.

Die EP 1 101 484 A2 beschreibt Füllstoffe für Dentalkomposite. Die Füllstoffe sind nicht für Verblendungen von dentalen Gerüsten geeignet.

Die in der DE 30 34 374 A1 beschriebenen Partikel werden als Füllstoffe in Dentalkomposite eingearbeitet. Diese Beschichtung besteht aus härtbaren (polymerisierbaren) Kunststoffen, vorzugsweise solchen aus der organischen Matrix des Dentaimaterials. Diese beschichteten Partikel sind als glasige oder glaskeramische Verblendung für Dentalrestauration nicht einsetzbar.

Die US 4,412,015 betrifft Füllstoffe für Dentalmaterialien primär auf der Basis von Bariumfluorsilikat-Gläsern bzw. Zeolithen. Die Beschichtung der Partikel mit härtbaren organischen Verbindungen dient ausschließlich der erhöhten Festigkeit des Verbundes zwischen den anorganischen Partikeln und der Polymermatrix.

In der WO 98/51419 A1 wird ein Partikelkern einer Sinterkeramik beschrieben, der von einer zweiten Metallverbindung umschlossen ist. Diese Beschichtung ist fähig, mit anderen Metallen eine feste Bindung einzugehen. Insgesamt sind diese Partikel den sintermetallurgischen Produkten zuzuordnen und mit den glasigen oder glaskeramischen Partikeln (Dentalpulver) nicht vergleichbar.

Die EP 1 250 895 A2 betrifft ein Verfahren zur Herstellung von dentalkeramischen Formteilen unter Anwendung der elektrophoretischen Abscheidung von oxidkeramischen Partikeln auf einem zahntechnischen Modell. Der beschriebene Schlicker wird elektrophoretisch auf ein Modell abgeschieden und der nach einem Trocknungsprozess so erhaltene oxidkeramische Grünkörper wird bei Temperaturen zwischen ca. 1100°C und etwa 1700°C dichtgesintert. Danach erfolgt die Verblendung auf aus dem Stand der Technik bekannte Art und Weise.

Die US 5,122,418 beschreibt die Herstellung eines Pulvers für kosmetische Anwendungen, wobei natürlich auch in diesem Falle bestimmte Eigenschaften im Vordergrund stehen.

Zusammenfassend ist festzustellen, dass in dem genannten Stand der Technik nicht eine Beschichtung aus Dentalpulver beschrieben wird, welche bei der Verarbeitung sowie thermischen Behandlung gute und vorteilhafte Verarbeitungseigenschaften aufweist.

Die Aufgabe der Erfindung besteht darin, eine Beschichtung, bevorzugt eine Nanobelegung, auf ein Dentalpulver aufzubringen, wodurch bei Verwendung einer Flüssigkeit, bevorzugt Wasser, ein solcher Schlicker entsteht, der gut verarbeitbar ist, eine optimale Fügung und Haftung zum Substrat erreicht wird, ohne dass eine Fügelücke auftritt. Weiterhin ist es das Ziel, optimale Schwindungsreaktionen von Dentalpulvem (Keramiken, Glaskeramiken , Gläsern) ohne Substrate zu erzielen.

Diese Aufgabe wird gelöst durch ein anorganisches Dentalpulver aus Gläsern oder Glaskeramiken auf alumino-silicatischer, alumino-phosphorsilicatischer oder alumino-borosilicatischer Basis, beschichtet mit einer Schicht enthaltend organische Stoffe und darauf einer Schicht enthaltend anorganische Stoffe, in einer Schichtdicke von 0,5 nm - 1 µm für die Verwendung als wässriger Schlicker.

Es wurde überraschend gefunden, dass ein mit organischen Komponenten belegtes anorganisches Pulver eine hohe Haftung der anorganischen Pulver auf der Unterlage ermöglichte und anorganische Komponenten, wie z.B. ZnCl₂ eine optimale Verarbeitung und Standfestigkeit der wässrigen Schlicker ermöglichen. Der zusätzlich überraschende Effekt bestand darin, dass beide, an sich entgegengesetzt wirkenden Mechanismen, durch die Belegung auf einem pulverförmigen Dentalwerkstoff zu realisieren waren.

Zur Charakterisierung des beschichteten Dentalpulvers wurde das Zetapotential bestimmt. Dabei zeigte es sich, dass das Zetapotential für verschiedene Dentalpulver aus Gläsern, Glaskeramiken und /oder Keramiken im Bereich von +100 bis -150, vorzugsweise im Bereich von +50 bis -80 liegt. Die Messung erfolgt dabei gegen einen Hintergrundelektrolyten, der vorzugsweise die gleiche anorganische Komponente ist wie in der Lösung zur Beschichtung des Dentalpulvers.

Die erfindungsgemäß eingesetzten Pulver sind vorzugsweise kristallin, glaskeramisch, glasig oder amorph. Als Dentalpulver kommen Gläser, oder Glaskeramiken auf alumino-silicatischer, alumino-phosphosilicatischer, oder alumino-borosilicatischer Basis in Betracht. Derartige Stoffsysteme enthalten z.B.: SiO₂-Al₂O₃-Na₂O-K₂O; oder SiO₂-Al₂O₃-Na₂O-K₂O-CaO-P₂O₅; oder SiO₂-Li₂O.

Die Glaskeramiken können Hauptkristallphasen enthalten, wie z.B. Leucit, oder Apatit, oder Leucit und Apatit.

Die Sinterkeramiken können silicatische Werkstoffe enthalten, z.B. Silicatgläser und Feldspatkristalle, oder Aluminiumoxid oder Zirkoniumoxid als Hauptbestandteil.

Die Korngröße der verwendeten Dentalpulver der Glaskeramiken, glashaltigen Sinterkeramiken oder glashaltigen Composite mit und ohne Kristallphasen kann bevorzugt zwischen 5 und 100 µm, vorzugsweise zwischen 5 und 50 µm (gemessen als mittlere Korngröße D₅₀) liegen. Für Dentalsinterkeramiken liegt diese im Bereich zwischen 50 nm und ca. 2 µm.

Die Schichtdicke der aufgebrachten organischen und anorganischen Substanzen beträgt 0,5 nm bis etwa 1 µm, vorzugsweise zwischen 0,5 und 200 nm.

Zur Beschichtung von Dentalpulvern (Gläsern, Glaskeramiken oder Keramiken) mit organischen Komponenten eignen sich insbesondere maßgeschneiderte Polymere, die aufgrund ihrer Struktur eine spezifische Wechselwirkung mit Festkörperoberfläche der Dentalpulver gestatten und damit infolge der Modifizierung bzw. Belegung der Pulveroberfläche zu entsprechenden Eigenschaftsänderungen führen. Geeignete Polymere sind vorzugsweise wasserlösliche Verbindungen, die für die Wechselwirkung mit der Festkörperoberfläche befähigte funktionelle Gruppen tragen, wobei die Wechselwirkung über Dipol-Dipol-, Ionen-Dipol, Ionen-Ionen-Wechselwirkungen sowie über Wasserstoffbrücken-Bindungen und Komplexierung erfolgen kann. Die Polymermodifizierung der Partikel führt dann zu solchen bei Polymer-Feststoff-Dispersionen bekannten Mechanismen wie sterische Stabilisation, Brückeneffekt oder Separation. Typisch wasserlösliche Polymere sind vor allem solche, die linear aufgebaut sind und eine große Anzahl von hydrophilen Gruppen enthalten. Dabei lassen sich die hydrophilen Gruppen in nichtionische (z.B. -OH, -O-, -NH₂, -NHR, -NR₂, -SH, -CO-NH-, -O-CO-NH-, -NH-CO-NH₂, -COOH, -P(O)(OH)₂), anionische (-COO⁻, -PO₃²⁻, -SO₃²⁻), kationische (NH₃⁺, -NR₃⁺), zwitterionische Gruppen (-NR₂⁺-O⁻, -NR₂⁺-(CH₂)ₙ-SO₃⁻) und hybride Makromoleküle unterteilen. Zu den wasserlöslichen Polymeren zählen solche synthetischen Polymeren wie Polyvinylalkohol, Polyethylenimin, Polyacrylamid, Polyethylenoxid, Polyethylenglycol, Homo- und Copoly-mere der (Meth)acrylsäure, Maleinsäure, Vinylsulfonsäure-und Vinylphosphonsäure, Polyvinylpyrrolidon sowie solche Biopolymere wie Stärke, Alginate, Gelatine oder Celluloseether, wie z.B. Carboxymethylcellulose.

Dabei eignen sich neben den Homopolymeren auch Copolymere mit maßgeschneiderten Struktur, die eine optimale Wechselwirkung mit der Festköperoberfläche gestatten. Es kann die Copolymerzusammensetzung der Reaktivität der Pulveroberfläche angepasst werden, so dass eine starke Wechselwirkung der Monomerbausteine der Makromoleküle mit den reaktiven Zentren der Pulver möglich wird. Beispielsweise fördern mehr saure Monomerbausteine eine intensivere ionische Wechselwirkung mit überwiegend basischen Oberflächen oder ermöglichen Monomerbausteine die Heteroatome (N, O) enthalten Wasserstoffbrückenbindungen mit oberflächlichen OH-Gruppen der Festkörperpulver. Darüber hinaus lassen sich gezielt chelatisierende Monomerbausteine, wie z.B. Bausteine mit Diketon- oder Salicylat-Gruppen, einbauen, die eine koordinationschemische Wechselwirkung mit vor allem Übergangsmetallatomen, wie z.B. Ti oder Zr, gestatten. Schließlich kann auch über die Architektur der Copolymeren die Dichte der Belegung der Partikeloberfläche variiert werden. So ist bekannt, dass z.B. maßgeschneiderte Block- und Kammpolymere sich im besonderen zur Oberflächenmodifizierung von Festkörperoperflächen eignen. Als steuerbare Strukturparameter kommen u.a. die Art der Monomerbausteine der Polymersegmente, die Segmentlänge und die Segmentverteilung in Frage.

Die Beschichtung der Dentalpulver kann bevorzugt durch zwei Methoden erfolgen:
1. Durch Belegung der Pulver mit Molekülen nach dem Prinzip der Selbstorganisation von Teilchen auf Pulveroberflächen während der Reaktion in wässrigen Medien.
2. Durch spezifische Beschichtungsverfahren, bevorzugt durch Wirbelbettbehandlung der Pulver.

Zum ersten Verfahren:
In einem oder mehreren Reaktionsschritten werden organische und / oder anorganische Komponenten aufgebracht. Im mehrstufigen Prozess wird z.B. so vorgegangen, dass eine bestimmter Stoffmengenanteil an einer organischen Komponente in destilliertes Wasser eingebracht wird und das Dentalpulver anschließend dazugegeben wird und eine Zeit von ca. 1 Minute bis 1 Stunde durch z.B.
Rühren homogenisiert wird. Durch Selbstorganisation erfolgt die Belegung des Dentalpulvers mit der organischen Komponente. Danach wird die Flüssigkeit vom Pulver getrennt (z.B. Filtrieren) und das Pulver für 10 Minuten bis 15 Stunden bei 80 bis 200°C getrocknet. Evtl. geringfügig auftretende Verklumpungen werden durch Schütteln oder leichtes Zerdrücken oder Sieben zerstört. Im zweiten Reaktionsschritt wird eine zweite wässrige Flüssigkeit mit anorganischen Komponenten versetzt / dotiert und das bereits mit der organischen Komponente belegte Pulver dazugegeben, homogenisiert, getrocknet und gemäß dem ersten Reaktionsschritt aufbereitet.

Ein Gegenstand der vorliegenden Erfindung ist mithin ein Verfahren zur Herstellung eines anorganischen Dentalpulvers für die Verwendung als wässriger Schlicker, gemäß obiger Beschreibung, dadurch gekennzeichnet, dass
I.
   (1) eine organische Komponente in destilliertes Wasser eingebracht wird,
   (2) Dentalpulver zugesetzt,
   (3) homogenisiert und
   (4) die Flüssigkeit vom Pulver getrennt wird, und
II.
   (1) eine zweite wässrige Flüssigkeit mit anorganischen Komponenten versetzt
   (2) das im Schritt I mit der organischen Komponente belegte Pulver zugesetzt,
   (3) homogenisiert und
   (4) getrocknet wird.

Das zweite Verfahren ist dadurch charakterisiert, dass z.B. in einer Wirbelbett-Anlage, der Fa. Glatt, Schweiz, eine Belegung mit organischen und anorganischen Komponenten erfolgt.

Als Ausgangsmaterial für die nachfolgenden Ausführungsbeispiele wurde eine Leucit-Apatit-Glaskeramik mit einer mittleren Korngröße D₅₀ von ca. 30 µm verwendet.

Für den Vergleich der einzelnen Dentalpulver sind drei verschiedene Arten sinnvoll denkbar:
1. unbeschichtetes Dentalpulver,
2. Dentalpulver beschichtet mit organischen (PEG 10.000) und anorganischen Substanzen (ZnCl₂), Beschichtung mittels Wirbelbett- Technologie nach Ausführungsbeispiel 2,
3. Dentalpulver beschichtet mit organischen (PEG 10.000) und anorganischen Substanzen (ZnCl₂) in wässriger Lösung, Beschichtung nach Ausführungsbeispiel 1 durchgeführt.

1. Unbeschichtetes Dentalpulver
   Im unbeschichteten Dentalpulver sind im REM-Bild isolierte Teilchen aller Größenordnungen zu erkennen, ggf. liegen einzelne kleine Partikel unsignifikant auf anderen, größeren Partikeln.
2. Wirbelbett - Technologie
   2.1 Die Schichtstärke der Beschichtung des Dentalpulvers mit den o.g. organischen und anorganischen Substanzen ist ganz offensichtlich unterhalb 1 µm, sehr wahrscheinlich sogar unterhalb 100 nm.
   2.2 Ein sehr großer Anteil der Partikel des Feinkornspektrums (Partikel kleiner als 25% der D₅₀, im genannten Beispielsfall also kleiner 5 µm) befindet sich auf der Oberfläche großer Partikel. Die Fixierung der Partikel ist relativ stark und lässt sich selbst durch eine direkte Bestrahlung mit einem 20 kV-Elektronenstrahl nicht aufbrechen und die kleinen Partikel von der Oberfläche entfernen.
   2.3 Weder innerhalb des Dentalpulvers noch auf der Oberfläche agglomerieren die kleinen Partikel zu größeren Kornansammlungen (Bildung von Aggregaten) zusammen, wie dies z.B. bei der Herstellung von ZrO₂- oder Al₂O₃- Keramiken bekannt ist.
3. Flüssigkeitstechnologie
   Auch bei dieser Technologie treffen die für unter 2. gemachten Aussagen zu, allerdings ist die Zahl der kleinen Partikel auf den großen etwas kleiner, was sicher auf die unterschiedlichen Verfahren zurückzuführen ist.

Aus den REM- Untersuchungen lassen sich nun folgende Schlussfolgerungen im Vergleich zur zahntechnischen Präparation einer Glaskeramikschicht auf einem Dentalsubstrat durch thermische Behandlung ziehen:
Es ist bekannt, dass der Schwindungsvorgang des Dentalpulvers während der thermischen Behandlung bis ca. 900°C durch verschiedene Parameter beeinflusst wird. Überraschend wurde festgestellt, dass durch die spezielle Packungsdichte, die durch die größeren Partikel, die mit kleinen behaftet sind, einen besonderen Vorteil auf die Schwindung hat.
Der Schwindungsvorgang im Zusammenhang mit der Freisetzung flüchtiger Bestandteile (Flüssigkeit des Schlickers, Freiwerden der eingeschlossenen Luft etc.) ist mit dem erfindungsgemäßen Dentalpulver deutlich geringer als mit unbeschichteten Dentalpulver.
Das Ergebnis der Versuche zeigt, dass die physikalisch-chemischen Reaktionen der Schwindung, der Freisetzung flüchtiger gasförmiger Bestandteile und der Aktivierung der Partikel im so genannten Niedertemperaturbereich (nicht nur unterhalb der Sintertemperatur, sondern sogar auch unterhalb des Transformationsbereiches der Glasmatrix der verwendeten Leucit-Apatit-Glaskeramik) von besonderer Bedeutung sind und durch das erfindungsgemäße Dentalpulver kein Verziehen der gewünschten Geometrie oder Abheben vom Substrat bis zur Sintertemperatur auftritt.
Während des Sintervorganges sind die kleinen Partikel des Feinkornspektrums des Dentalpulvers von besonderer Bedeutung. Diese kleinen Partikel besitzen im Vergleich zu den größeren eine höhere Sinteraktivität und leiten den Sintervorgang ein. Gleichzeitig ermöglichen diese durch ihre schneller auftretende Liquidität die Herstellung einer kompakten und porenarmen Dentalrestauration (vgl. Abbildung).

### Ausführungsbeispiel

### Selbstorganisation von Monolagen organischer und anorganischer Stoffe auf anorganischen Pulvern in wässrigen Lösungen

### Beschichten einer Leucit-Apatit-Glaskeramik mit PEG und ZnCl₂:

Zur Beschichtung von 60 g Glaskeramikpulver der mittleren Korngröße D₅₀ von 32 µm wurden 26.58 g PEG-Lösung (Gehalt an PEG: 1,0 Ma.-%) benötigt.

Diese Mengen an Pulver und Liquid wurden in einem Vorversuch ermittelt, bei dem zur Beschichtung einer dreigliedrigen Dental-Metall-Brücke mit 5.53 g Pulver und 2.45 g Anmischflüssigkeit vorgenommen wurden. Die 60 g Glaskeramik wurden in einem Becherglas eingewogen, die 26.58 g der Lösung zugegeben und mit destilliertem Wasser auf 100 g aufgefüllt. Nachfolgend wurde 20 Minuten gerührt. Anschließend erfolgte das Trennen der Flüssigkeit vom Pulver durch Filtrieren über eine Glassinternutsche und eine Saugflasche bei geringem Vakuum. Der Filterkuchen wurde abgeschöpft und bei 80°C und 12 Stunden Haltezeit getrocknet. Wenige entstandene kleine Verklumpungen werden durch Andrücken zerkleinert.

Von diesem mit organischen Molekülen belegten Dentalpulver wurde anschließend das Zetapotential mit der Apparatur ESA 8000, Fa. Matec appl. Sci., Northborough, MA, USA gemessen. Als Hintergrundelektrolyt wurde eine ZnCl₂-Lösung verwendet. Im vorliegenden Ausführungsbeispiel wurde eine Suspension mit 8 Vol.-% Feststoffgehalt hergestellt. Dies entspricht 50.8 g Pulver + 230 ml Lösung, wobei als Hintergrundelektrolyt eine Zinkchlorid- Lösung verwendet wurde. Unter Rühren erfolgte die Zugabe von 1M ZnCl₂- Lösung. Es erfolgte eine Nanobelegung mit anorganischen Molekülen auf der Oberfläche des bereits mit organischen Molekülen belegten Dentalpulvers. Beide Prozesse der Nanobelegung erfolgten nach den Prinzipien der Selbstorganisation. Als letzter Prozessschritt der Pulverbelegung wurde filtriert, getrocknet und aufbereitet.

Ergebnisse der Zetapotentialmessung:
1. Pulver: Leucit-Apatit-Glaskeramk belegt mit PEG-6000
a) entspricht: Pulver mit PEG belegt vor ZnCl₂-Belegung
b) entspricht: Pulver mit PEG belegt nach ZnCl₂-Belegung

| | |
|---|---|
| a) PEG-6000 2Ma%: | -73.8 mV ζ-Potential |
| b) + 2.3 ml ZnCl₂ 1M: | +25.7 mV ζ-Potential |

2. Pulver: Leucit-Apatit-Glaskeramik belegt mit PEG-10000

| | |
|---|---|
| a) PEG 10000 1,0 Ma.-%: | -64.6 mV ζ-Potential |
| b) + 1,7 ml ZnCl₂ 1M: | -20.6 mV ζ-Potential |

Diese Dentalpulver mit Nanobelegung wurden im zahntechnischen Versuch auf Metallgerüste, die mit einem Opaker beschichtet waren, aufgebracht. Dabei wurde so

Vorgegangen dass aus den Pulvern durch Zugabe von destillierten Wassers ein gebrauchsfertiger Schlicker erzeugt wurden. Diese Schlicker wurden auf viergliedrige, mit Opaker beschichtete Metall- Brücken aufgebracht. Die thermische Behandlung wurde im Ofen P 100, Fa. Ivoclar Vivadent AG unter folgenden Parametern vorgenommen:
- Aufheizrate: 60 K/min von Raumtemperatur bis 890°C
- Vakuum ab 400°C bis 890°C
- Haltezeit bei 890°C: 1 min

Nach Abkühlung der Dentalrestaurationen zeigte sich, dass die Benetzung der aufgebrachten nanobelegten Glaskeramik mit dem Gerüst in cervicalen Bereich der Dentalbrücke sowohl für die Varianten mit PEG-6000, wie auch mit PEG-10000 gut war. Der Probentyp mit PEG-10000 zeigte allerdings das beste Reaktionsverhalten in Bezug auf den Gesamt-Fügeverbund und die Schwindung der Dentalkeramik.

Gleichfalls wurden nach diesem Verfahren Probeplättchen hergestellt und deren optische Parameter bestimmt. Es zeigte sich im Vergleich zu einer unbeschichteten/ unbelegten Probe keinerlei Verfärbung durch Kohlenstoffreste. Im Sinterprozess sind die organischen Bestandteile bis 890°C restlos ausgebrannt worden.

Details zum Ausbrennvorgang zeigt Ausführungsbeispiel 2.

### Ausführungsbeispiel: Wirbelbettbeschichtung

Als Ausgangsmaterial wurde 1 kg des gleichen Leucit-Apatit-Glaskeramikpulvers wie in Beispiel verwendet.

Dieses Pulver wurde mit einer Lösung des Gehaltes an 1,0 Ma.-% PEG 1000 beschichtet. Zur Beschichtung wird der GLATT Wirbelschichtgranulierer GPC G1.1 (Hersteller Fa. Glatt, Schweiz) verwendet.

Das Pulver wird in den Trichter der Glatt-Anlage eingefüllt, der Schlauch der Förderpumpe wird in die PEG Lösung eingetaucht.

Die Parameter werden eingestellt mussten jedoch während des Beschichtungsvorgangs angepasst werden. Es war darauf zu achten, dass die Liquidmenge und Zulufttemperatur so eingestellt wurden, dass das Pulver nicht verklumpt.

### Versuchsparameter:

| | |
|---|---|
| Zulufttemp.: | 35 - 40°C |
| Sprühdruck: | 1 bar |
| Pumpenpositon: | 30 - 35% |
| Versprühte Menge: | ca. 14.2 ml/ min |
| Luftmenge: | 80 - 120 m³/h |
| Dauer Beschichtung: | 70.5 min |
| Trocknungsdauer: | bis Produkttemperatur von 24°C erreicht wird |

Im Ergebnis dieses Versuches wurde das beschichtete Glaskeramikpulver in einem thermischen Versuch mittels Thermogravimetrie untersucht. Die Auswertung diese Versuches zeigte, dass im Bereich von ca. 350 bis 400°C ein Masseverlust von ca. 1,0 Ma.-% auftrat, was dem Gehalt an PEG-1000 entspricht, denn in diesem Temperaturbereich zersetzt sich das PEG.

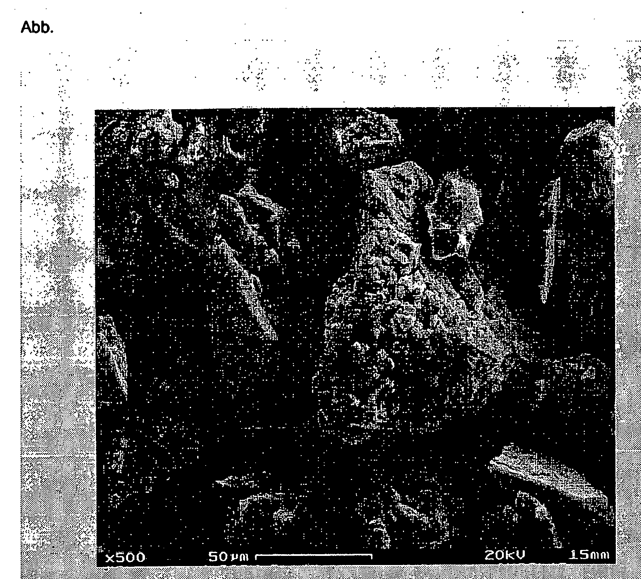

## Patentansprüche

1. Anorganisches Dentalpulver aus Gläsern oder Glaskeramiken auf alumino-silicatischer, alumino-phosphorsilicatischer oder alumino-borosilicatischer Basis, beschichtet mit einer Schicht enthaltend organische Stoffe und darauf einer Schicht enthaltend anorganische Stoffe, in einer Schichtdicke von 0,5 nm - 1 µm für die Verwendung als wässriger Schlicker.

2. Anorganisches Dentalpulver nach Anspruch 1, **dadurch gekennzeichnet, dass** die Schichtdicke 0,5 - 200 nm beträgt.

3. Dentalpulver nach einem der vorhergehenden Ansprüche, **dadurch gekennzeichnet, dass** die durchschnittliche Korngröße 50 nm bis 100 µm beträgt.

4. Dentalpulver nach einem der vorhergehenden Ansprüche, **dadurch gekennzeichnet, dass** die durchschnittliche Korngröße 5 - 50 µm beträgt.

5. Dentalpulver nach einem der vorhergehenden Ansprüche, **dadurch gekennzeichnet, dass** es eine oder mehrere Kristallphasen enthält.

6. Dentalpulver nach einem der vorhergehenden Ansprüche, **dadurch gekennzeichnet, dass** es als Kristallphasen Leucit oder Fluor-Apatit oder Gemische hiervon enthält.

7. Dentalpulver nach einem der vorhergehenden Ansprüche, **dadurch gekennzeichnet, dass** das Zetapotential eines durch Zusatz von Wasser zu dem Pulver erzeugten Schlickers +100 bis -150 mV, vorzugsweise +50 bis -80 mV beträgt.

8. Dentalpulver nach einem der vorhergehenden Ansprüche, **dadurch gekennzeichnet, dass** deren Beschichtung als anorganische Stoffe ZnCl₂ oder AlCl₃ enthält.

9. Dentalpulver nach einem der vorhergehenden Ansprüche, **dadurch gekennzeichnet, dass** deren Beschichtung als organische Stoffe wasserlösliche Polymere enthält.

10. Dentalpulver nach Anspruch 9, **dadurch gekennzeichnet, dass** die Polymere Verbindungen mit hydrophilen Gruppen oder hybride Makromoleküle sind.

11. Dentalpulver nach Anspruch 10, **dadurch gekennzeichnet, dass** die hydrophilen Gruppen nichtionische, anionische, kationische oder zwitterionische Gruppen sind.

12. Dentalpulver nach Anspruch 9 bis 11, **dadurch gekennzeichnet, dass** es synthetische Polymere oder Biopolymere enthält.

13. Dentalpulver nach Anspruch 12, **dadurch gekennzeichnet, dass** die synthetischen Polymere Polyvinylalkohol, Polyethylenimin, Polyethylenoxid, Polyethylenglycol, Homo- und Copolymere der (Meth)acrylsäure, Maleinsäure, Vinylsulfonsäure- und Vinylphosphonsäure, Polyvinylpyrrolidon und die biopolymere Stärke, Alginate, Gelatine oder Celluloseether, vorzugsweise Carboxymethylcellulose sind.

14. Dentalpulver nach Anspruch 12 oder 13, **dadurch gekennzeichnet, dass** die organischen Polymere Block- und Kammpolymere bzw. Homo- und Copolymere mit chelatisierenden Monomerbausteinen sind.

15. Verfahren zur Herstellung eines anorganischen Dentalpulvers für die Verwendung als wässriger Schlicker, gemäß einem der vorhergehenden Ansprüche, **dadurch gekennzeichnet, dass**
I.
(1) eine organische Komponente in destilliertes Wasser eingebracht wird,
(2) Dentalpulver zugesetzt,
(3) homogenisiert und
(4) die Flüssigkeit vom Pulver getrennt wird, und
II.
(1) eine zweite wässrige Flüssigkeit mit anorganischen Komponenten versetzt
(2) das im Schritt I mit der organischen Komponente belegte Pulver zugesetzt,
(3) homogenisiert und
(4) getrocknet wird.

16. Verfahren nach Anspruch 15, **dadurch gekennzeichnet, dass** das Homogenisieren durch Rühren erfolgt.

17. Verfahren nach einem der vorhergehenden Ansprüche, **dadurch gekennzeichnet, dass** die Auftrennung von Pulver und Flüssigkeit mittels Filtrierens erfolgt.

18. Verfahren nach einem der vorhergehenden Ansprüche, **dadurch gekennzeichnet, dass** die Trocknung bei 80°C bis 200°C durchgeführt wird.

19. Verfahren nach einem der vorhergehenden Ansprüche, **dadurch gekennzeichnet, dass** die Dispersion der anorganischen und organischen Komponenten mittels eines Wirbelbettverfahrens auf das Pulver aufgebracht werden.

20. Verwendung des Dentalpulvers nach einem der Ansprüche 1 bis 14 zur Herstellung von metallfreien und/oder metallgestützten Dentalrestaurationen wie Brücken, Kronen, Teilkronen.

## Claims

1. An inorganic dental powder consisting of glass or glass ceramics based on an aluminosilicate, aluminophosphosilicate, or aluminoborosilicate coated with a layer comprising organic substances and thereupon a layer comprising inorganic substances in a layer thickness of 0.5 nm - 1 µm for the use as an aqueous slip.

2. The inorganic dental powder as claimed in claim 1, wherein the layer thickness is 0.5 - 200 nm.

3. The dental powder as claimed in one of the preceding claims, wherein the average particle size is 50 nm to 100 µm.

4. The dental powder as claimed in one of the preceding claims, wherein the average particle size is 5 - 50 µm.

5. The dental powder as claimed in one of the preceding claims, which contains one or more crystal phases.

6. The dental powder as claimed in one of the preceding claims, which as crystal phases contains leucite or fluoroapatite or mixtures thereof.

7. The dental powder as claimed in one of the preceding claims, wherein the zeta potential of a slip, generated by the additon of water to the powder, is +100 to -150 mV, preferably +50 to -80mV.

8. The dental powder as claimed in one of the preceding claims, wherein its coating contains ZnCl₂ or AlCl₃ as inorganic substances.

9. The dental powder as claimed in one of the preceding claims, wherein its coating contains water-soluble polymers as organic substances.

10. The dental powder as claimed in claim 9, wherein the polymers are compounds having hydrophilic groups or hybrid macromolecules.

11. The dental powder as claimed in claim 10, wherein the hydrophilic groups are nonionic, anionic, cationic or zwitterionic groups.

12. The dental powder as claimed in claim 9 to 11, which contains synthetic polymers or biopolymers.

13. The dental powder as claimed in claim 12, wherein it the synthetic polymers are polyvinyl alcohol, polyethylenimine, polyethylene oxide, polyethylene glycol, homo- and copolymers of (meth)acrylic acid, maleic acid, vinylsulfonic acid and vinylphosphonic acid, polyvinyl-pyrrolidone and biopolymeric starch, alginates, gelatin or cellulose ethers, preferably carboxymethylcellulose.

14. The dental powder as claimed in claim 12 or 13, wherein the organic polymers are block and comb polymers or homo- and copolymers having chelating monomer units.

15. A process for the production of the inorganic dental powders, as claimed in one of the preceding claims, which comprises
I.
(1) introducing an organic component into distilled water
(2) adding dental powder,
(3) homogenizing and
(4) separating the liquid from the powder, and
II.
(1) treating a second aqueous liquid with inorganic components
(2) adding the powder coated with the organic component in step I,
(3) homogenizing and
(4) drying.

16. The process as claimed in claim 15, wherein the homogenization is carried out by stirring.

17. The process as claimed in one of the preceding claims, wherein the separation of powder and liquid is carried out by means of filtering.

18. The process as claimed in one of the preceding claims, wherein the drying is carried out at 80°C to 200°C.

19. The process as claimed in one of the preceding claims, wherein the dispersion of the inorganic and organic components is applied to the powder by means of a fluidized bed process.

20. The use of the dental powder as claimed in one of claims 1 to 14 for the production of metal-free and/or metal-supported dental restorations such as bridges, crowns and partial crowns.

## Revendications

1. Poudre dentaire inorganique en verres ou céramiques de verre à base aluminosilicatée, aluminophosphorosilicatée ou aluminoborosilicatée, revêtus par une couche contenant des substances organiques et, sur celle-ci, par une couche contenant des substances inorganiques, en une épaisseur de couche de 0,5 nm-1 µm, destinée à l'utilisation comme barbotine aqueuse.

2. Poudre dentaire inorganique selon la revendication 1, **caractérisée en ce que** l'épaisseur de couche est de 0,5-200 nm.

3. Poudre dentaire selon l'une quelconque des revendications précédentes, **caractérisée en ce que** la grosseur moyenne des grains est de 50 nm à 100 µm.

4. Poudre dentaire selon l'une quelconque des revendications précédentes, **caractérisée en ce que** la grosseur moyenne des grains est de 5-50 µm.

5. Poudre dentaire selon l'une quelconque des revendications précédentes, **caractérisée en ce qu'**elle contient une ou plusieurs phases cristallines.

6. Poudre dentaire selon l'une quelconque des revendications précédentes, **caractérisée en ce qu'**elle contient, comme phase cristalline, de la leucite ou de la fluoroapatite ou des mélanges de celles-ci.

7. Poudre dentaire selon l'une quelconque des revendications précédentes, **caractérisée en ce que** le potentiel Zeta d'une barbotine produite par addition d'eau à la poudre vaut +100 à -150 mV, de préférence +50 à -80 mV.

8. Poudre dentaire selon l'une quelconque des revendications précédentes, **caractérisée en ce que** son revêtement contient, comme substances inorganiques, ZnCl₂ ou AlCl₃.

9. Poudre dentaire selon l'une quelconque des revendications précédentes, **caractérisée en ce que** son revêtement contient, comme substances organiques, des polymères solubles dans l'eau.

10. Poudre dentaire selon la revendication 9, **caractérisée en ce que** les polymères sont des composés présentant des groupes hydrophiles ou des macromolécules hybrides.

11. Poudre dentaire selon la revendication 10, **caractérisée en ce que** les groupes hydrophiles sont des groupes non ioniques, anioniques, cationiques ou zwittérioniques.

12. Poudre dentaire selon la revendication 9 à 11, **caractérisée en ce qu'**elle contient des polymères synthétiques ou des biopolymères.

13. Poudre dentaire selon la revendication 12, **caractérisée en ce que** les polymères synthétiques sont un poly(alcool vinylique), une polyéthylèneimine, un poly(oxyde d'éthylène), un polyéthylèneglycol, des homopolymères et copolymères de l'acide (méth)acrylique, de l'acide maléique, de l'acide vinylsulfonique et de l'acide vinylphosphonique, une polyvinylpyrrolidone et les biopolymères sont l'amidon, un alginate, une gélatine ou un éther de cellulose, de préférence la carboxyméthylcellulose.

14. Poudre dentaire selon la revendication 12 ou 13, **caractérisée en ce que** les polymères organiques sont des polymères séquencés et des polymères à peigne ou des homopolymères et des copolymères présentant des éléments monomères chélatants.

15. Procédé pour la préparation d'une poudre dentaire inorganique destinée à être utilisée comme barbotine aqueuse selon l'une quelconque des revendications précédentes, **caractérisé en ce que**
I. (1) on introduit un composant organique dans de l'eau distillée,
(2) on ajoute de la poudre dentaire,
(3) on homogénéise et
(4) on sépare le liquide de la poudre, et
II. (1) on ajoute des composants inorganiques à un deuxième liquide aqueux
(2) on ajoute la poudre revêtue des composants organiques dans l'étape I,
(3) on homogénéise et
(4) on sèche.

16. Procédé selon la revendication 15, **caractérisé en ce que** l'homogénéisation est réalisée par agitation.

17. Procédé selon l'une quelconque des revendications précédentes, **caractérisé en ce que** la séparation de la poudre et du liquide a lieu au moyen d'une filtration.

18. Procédé selon l'une quelconque des revendications précédentes, **caractérisé en ce que** le séchage est réalisé à 80°C jusqu'à 200°C.

19. Procédé selon l'une quelconque des revendications précédentes, **caractérisé en ce que** la dispersion des composants inorganiques et organiques est appliquée sur la poudre au moyen d'un procédé en lit fluidisé.

20. Utilisation de la poudre dentaire selon l'une quelconque des revendications 1 à 14 pour la réalisation de réparations dentaires exemptes de métal et/ou soutenues par du métal, telles que des bridges, des couronnes, des couronnes partielles.
